# EUROPEAN PATENT APPLICATION

(11) **EP 4 606 893 A1**
(43) Date of publication of application: **27.08.2025**
(21) Application number: 23898063.5
(22) Date of filing: 13.10.2023
(51) Int. Cl.: C12N 9/02, C12N 15/70, C12P 7/52, C12P 7/625

(54) **NOVEL VARIANT POLYPEPTIDE HAVING PROPIONALDEHYDE DEHYDROGENASE ACTIVITY AND USE THEREOF**

(30) Priority: 29.11.2022 KR 20220163158
(71) Applicant: CJ CheilJedang Corporation, Seoul 04560 (KR)
(72) Inventor: KOO, A Young, Seoul 04560 (KR); LEE, Imsang, Seoul 04560 (KR); JEON, Ae Ji, Seoul 04560 (KR); PARK, Seong-Hee, Seoul 04560 (KR); LEE, Ji Sun, Seoul 04560 (KR)
(74) Representative: Meissner Bolte Partnerschaft mbB
(86) International application number: PCT/KR2023/015814
(87) International publication number: WO 2024/117529

(57) **Abstract**

The present disclosure relates to a novel variant polypeptide having propionaldehyde dehydrogenase activity; a polynucleotide encoding the variant polypeptide; a microorganism comprising the variant polypeptide, a polynucleotide encoding the variant polypeptide, or a vector containing the polynucleotide; and a method for producing 3HP-CoA or derivatives thereof, comprising the step of culturing the microorganism in a medium.

## Description

### [Technical Field]

The present disclosure relates to a variant polypeptide having propionaldehyde dehydrogenase activity; a polynucleotide encoding the variant polypeptide; a microorganism including the variant polypeptide, a polynucleotide encoding the variant polypeptide, or a vector containing the polynucleotide; and a method for producing 3HP-CoA or derivatives thereof, including culturing the microorganism in a medium.

### [Background Art]

Acrylic acid (AA), which is a highly reactive substance having a double bond and a functional group within the molecule, is used as a monomer for production of polymers through various chemical reactions and thus is produced for various purposes such as acrylics, fibers, paints, *etc.* In particular, glacial AA with high purity is mainly used for production of superabsorbent polymers (SAP) and is commercialized as a moisture absorbent in diapers and sanitary products. Most AA is currently produced through oxidation reaction of petrochemical-based propylene, but due to recent environmental and temperature issues and an increase in consumer needs for bio-friendly chemical products, the need for production of acrylic acid through bio-based routes other than petroleum-based routes is increasing.

In this regard, there have been various attempts to produce 3-hydroxypropionic acid (3HP), a precursor of AA, through genetic manipulation and fermentation of microorganisms. For example, examples may include a method of producing AA through a conversion reaction from lactic acid (LA) or 3HP, or a method of converting poly-3-hydroxypropionic acid (P3HP) into AA through thermal decomposition, *etc.* In the latter case, it has the advantage that AA is produced as a solid-state precursor, unlike 3HP, which is produced in a liquid state through purification from a dissolved state.

Meanwhile, 3HP can be produced biologically via an enzymatic reaction by 3HP-CoA dependent pathway and 3HP-CoA independent pathway using glycerol as a carbon source, which is produced as a by-product of biodiesel. 3HP is a useful intermediate that can be converted to various substances such as acrylic acid, acrylonitrile, methyl acrylic acid, 1,3-propanediol, *etc.*

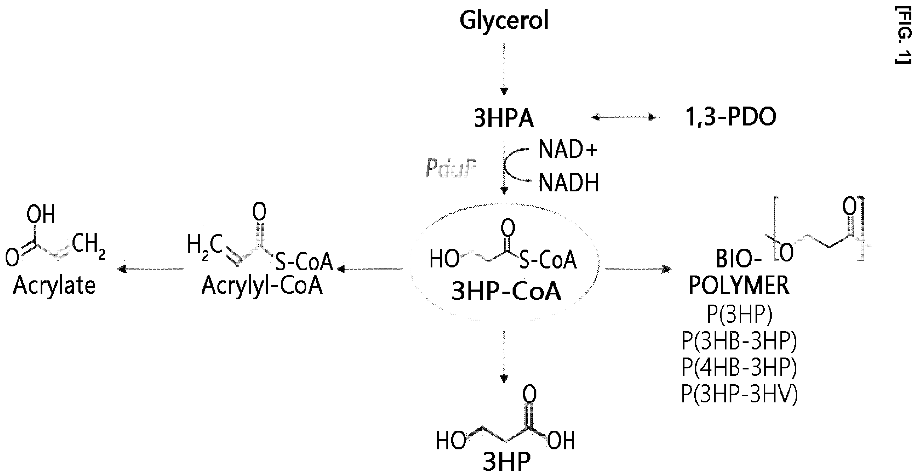

As shown in FIG. 1, the 3HP-CoA dependent pathway is an enzymatic reaction by which 3-hydroxypropionaldehyde (3-HPA) is converted to 3HP-CoA by the pduP enzyme. The produced 3HP-CoA can later be converted to AA, 3HP, or P3HP, *etc.*

Studies on the use of pduP to produce 3HP have been reported in previous literature (Bioresource Technology, Volume 103, Issue 1, January 2012, Pages 1-6). However, most of the previous studies only disclose the wild-type pduP derived from *Salmonella typhimurium* LT2 or *Lactobacillus reuteri* (Bioresour Technol. 2013 Mar;131:548-51.; KR 10-2016-0006030 A), and no studies on pduP mutants and their uses have been disclosed.

Therefore, studies to increase the production ability of effective 3HP-CoA or derivatives thereof are still needed.

### [Disclosure]

### [Technical Problem]

The technical problem to be solved in the present disclosure is to provide a variant polypeptide having novel propionaldehyde dehydrogenase activity; a polynucleotide encoding the variant polypeptide; a microorganism including the variant polypeptide, a polynucleotide encoding the variant polypeptide, or a vector containing the polynucleotide; and a method for producing 3HP-CoA or derivatives thereof, including culturing the microorganism in a medium.

### [Technical Solution]

One object of the present disclosure is to provide a variant polypeptide having propionaldehyde dehydrogenase activity in which the amino acid corresponding to position 272 of SEQ ID NO: 1 is substituted with another amino acid.

In one embodiment, the variant polypeptide may be one in which the amino acid corresponding to position 272 of SEQ **ID** NO: 1 is substituted with any one selected from the group consisting of glycine, alanine, arginine, valine, leucine, methionine, threonine, asparagine, glutamine, proline, serine, tryptophan, phenylalanine, histidine, cysteine, tyrosine, lysine, aspartate, and glutamic acid.

In another embodiment, the variant polypeptide may include an amino acid sequence selected from the group consisting of SEQ **ID** NOS: 3 to 7 and SEQ **ID** NOS: 33 to 46.

Another object of the present disclosure is to provide a polynucleotide encoding the variant polypeptide.

Still another aspect of the present disclosure is to provide a microorganism including any one or more of the variant polypeptide, a polynucleotide encoding the variant polypeptide, and a vector containing the polynucleotide.

In one embodiment, the microorganism has an increased production ability of 3HP-CoA or derivatives thereof, as compared to a microorganism including a polypeptide of SEQ ID NO: 1 or a polynucleotide encoding the polypeptide.

As the microorganism according to any one of the preceding embodiments, the microorganism may be a microorganism of the genus *Escherichia.*

As the microorganism according to any one of the preceding embodiments, the microorganism of the genus *Escherichia* may be *Escherichia coli.*

Still another object of the present disclosure is to provide a method for producing 3HP-CoA or derivatives thereof, including culturing the microorganism in a medium.

In one embodiment, the method may further include a step of recovering a target material from the cultured microorganism, a culture product of the microorganism, a fermented product of the microorganism, or the culture medium.

Still another object of the present disclosure is to provide a composition for producing 3HP-CoA or derivatives thereof, including the variant polypeptide, the microorganism, or a combination thereof.

Still another object of the present disclosure is to provide use of the microorganism for the production of 3HP-CoA or derivatives thereof.

### [Advantageous Effects]

When a microorganism including the variant polypeptide having propionaldehyde dehydrogenase activity of the present disclosure is cultured, 3HP-CoA or derivatives thereof can be produced at a higher yield, as compared to an existing non-modified microorganism.

### [Brief Description of Drawings]

FIG. 1 is a schematic diagram illustrating a pathway for producing 3HP-CoA or derivatives thereof from glucose or glycerol of the present disclosure.
FIG. 2 is a schematic diagram of the pFS-pduP(WT) plasmid.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Additionally, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

One aspect of the present disclosure provides a variant polypeptide having propionaldehyde dehydrogenase activity in which the amino acid corresponding to position 272 of SEQ ID NO: 1 is substituted with another amino acid.

As used herein, the term "variant polypeptide having propionaldehyde dehydrogenase activity" means a variant polypeptide having propionaldehyde dehydrogenase activity which includes one or more amino acid substitutions in an amino acid sequence of a polypeptide having propionaldehyde dehydrogenase activity.

As used herein, the term "variant polypeptide having propionaldehyde dehydrogenase activity" means a variant of a polypeptide having propionaldehyde dehydrogenase activity which includes one or more amino acid substitutions in the parent sequence, an amino acid sequence of a polypeptide having propionaldehyde dehydrogenase activity.

As used herein, the term "CoA-acylating propionaldehyde dehydrogenase (PduP)" means an enzyme which converts 3-hydroxypropionaldehyde (3HPA) into 3-hydroxypropionyl-CoA (3HP-CoA).

The gene encoding the CoA-acylating propionaldehyde dehydrogenase may be *pduP* derived from *Salmonella sp., Klebsiella pneumoniae, Lactobacillus,* specifically derived from *Salmonella enterica,* but is not limited thereto.

Specifically, the CoA-acylating propionaldehyde dehydrogenase may include, for example, an amino acid sequence of SEQ ID NO: 1, or an amino acid sequence having 60% or more homology or identity thereto, but is not limited as long as it has propionaldehyde dehydrogenase activity. Specifically, the amino acid sequence may include the amino acid sequence of SEQ ID NO: 1 or an amino acid sequence having at least 60%, 70%, 80%, 85%, 90%, 95%, 96%, 97%, 98%, or 99% or more homology or identity to SEQ ID NO: 1. The sequence of SEQ ID NO: 1 can be obtained from known database, NCBI's GenBank or Kyoto Encyclopedia of Genes and Genomes (KEGG). In one example, the amino acid sequence may be derived from *Salmonella* sp., or *Salmonella enterica,* and more specifically may be a polypeptide/protein including the amino acid sequence represented by SEQ ID NO: 1, but is not limited thereto. Additionally, it is apparent that any accessory protein having an amino acid sequence in which part of the sequence is deleted, modified, substituted, or added may fall within the scope of the present disclosure if the amino acid sequence has such a homology or identity and exhibits an efficacy corresponding to that of the protein.

Further, the CoA-acylating propionaldehyde dehydrogenase having the amino acid sequence of SEQ ID NO: 1 may be encoded by a polynucleotide, which may have or include a nucleotide sequence of SEQ ID NO: 2, or a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequence of SEQ ID NO: 2, or may consist or essentially consist of a nucleotide sequence of SEQ ID NO: 2, or a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequence of SEQ ID NO: 2, but is not limited thereto.

As used herein, the term "variant" refers to a polypeptide having one or more amino acids different from the amino acid sequence before mutation of the variant by conservative substitutions and/or modifications such that the functions and properties of the polypeptide are retained. Such variants may generally be identified by modifying one or more of the amino acid sequences of the polypeptide and evaluating the properties of the modified polypeptide. That is, the ability of the variants may be enhanced, unchanged or reduced relative to a polypeptide before mutation. Additionally, some variants may include those in which one or more regions, such as an N-terminal leader sequence or transmembrane domain, have been removed. Further, other variants may include those in which a region has been removed from the N- and/or C-terminal of a mature protein. The term "variant" may be used interchangeably with terms such as modification, modified polypeptide, modified protein, mutant, mutein, divergent, *etc.* without limitation, as long as the terms are used to indicate variation.

Additionally, the variant may also include deletion or addition of amino acids that have minimal influence on the properties and secondary structure of a polypeptide. For example, the polypeptide may be conjugated with a signal (or leader) sequence at the N-terminal involved in the transfer of proteins co-translationally or post-translationally. Further, the polypeptide may also be conjugated with another sequence or linker to identify, purify, or synthesize the polypeptide.

The variant polypeptide having propionaldehyde dehydrogenase activity of the present disclosure may be a variant polypeptide having propionaldehyde dehydrogenase in which the amino acid corresponding to position 272 of SEQ ID NO: 1 is substituted with another amino acid, but is not limited thereto.

The "substitution with another amino acid" is not limited as long as it is substituted with an amino acid other than the amino acid before substitution. Meanwhile, in the present disclosure, when it is expressed that 'a specific amino acid has been substituted', it is apparent that the amino acid is substituted with an amino acid different from the amino acid before substitution, even if it is not specifically stated that the amino acid has been substituted with a different amino acid.

The amino acids may generally be classified based on similarity of polarity, charge, solubility, hydrophobicity, hydrophilicity, and/or amphipathic nature of a residue.

The amino acids may be classified into the following groups:
In one example, positively charged (basic) amino acids include arginine, lysine, and histidine; negatively charged (acidic) amino acids include glutamic acid and aspartic acid; amino acids having nonpolar side chains (nonpolar amino acids) include glycine, alanine, valine, leucine, isoleucine, methionine, phenylalanine, tryptophan, and proline; amino acids having polar or hydrophilic side chains (polar amino acids) include serine, threonine, cysteine, tyrosine, asparagine, and glutamine. In another example, amino acids having electrically charged side chains (electrically charged amino acids) include arginine, lysine, histidine, glutamic acid, aspartate; and amino acids having uncharged side chains (referred to as uncharged amino acids; neutral amino acids) include glycine, alanine, valine, leucine, isoleucine, methionine, phenylalanine, tryptophan, proline, serine, threonine, cysteine, tyrosine, asparagine, and glutamine. In still another example, aromatic amino acids include phenylalanine, tryptophan, and tyrosine. In yet another example, branched amino acids include valine, leucine, and isoleucine. In even another example, the 20 amino acids can be classified according to their size into 5 groups, starting from the amino acid groups with a relatively small volume, i.e., glycine, alanine, serine; cysteine, proline, threonine, aspartate, asparagine; valine, histidine, glutamic acid, glutamine; isoleucine, leucine, methionine, lysine, arginine; and phenylalanine, tryptophan, and tyrosine, but the classification of the amino acids is not limited thereto.

For example, when it is described as "the amino acid corresponding to position 272 of SEQ ID NO: 1 is substituted with another amino acid", it may mean that the amino acid is substituted with glycine, alanine, glutamate, phenylalanine, arginine, aspartate, cysteine, asparagine, glutamine, histidine, proline, serine, tyrosine, lysine, tryptophan, valine, methionine, threonine, or leucine, except isoleucine, but is not limited thereto.

In the present disclosure, although it is described as "a protein having an amino acid sequence described by a specific sequence number", it is apparent that any protein having an amino acid sequence in which part of the sequence is deleted, modified, substituted, conservatively substituted or added can be used in the present disclosure if it has the same or corresponding activity as the protein consisting of the amino acid sequence of the corresponding sequence number. For example, sequence additions upstream or downstream of the amino acid sequences that do not alter the function of the protein, naturally occurring mutations, silent mutations thereof, or conservative substitutions are not excluded, as long as the protein has activity identical or corresponding to the activity of the variant protein, and it will be apparent to those skilled in the art that such sequence additions or mutations belong to the scope of the present disclosure.

The "N^{th} position" of the present disclosure may include the N^{th} position and an amino acid position corresponding to the N^{th} position. Specifically, it may include an amino acid position corresponding to any amino acid residue in a mature polypeptide disclosed in a specific amino acid sequence. The specific amino acid sequence may be the amino acid sequence of SEQ ID NO: 1.

As used herein, the term "corresponding to" refers to an amino acid residue at the position recited in a peptide, or an amino acid residue which is similar, identical, or homologous to the residue recited in a peptide. Identifying an amino acid at a corresponding position may be determining a particular amino acid in a sequence that refers to a particular sequence. As used herein, the "corresponding region" generally refers to a similar or corresponding position in the related protein or reference protein.

For example, any amino acid sequence is aligned with SEQ ID NO: 1, and based on the alignment, each amino acid residue of the amino acid sequence can be numbered with reference to the numerical position of the amino acid residue corresponding to the amino acid residue of SEQ ID NO: 1. For example, a sequence alignment algorithm such as that described herein can identify the position of an amino acid or a position where modifications such as substitutions, insertions or deletions occur compared to a query sequence (also referred to as a "reference sequence").

Example of the alignment may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277), *etc.,* but is not limited thereto, and sequence alignment programs, such as pairwise sequence comparison algorithms, *etc.,* known in the art may be appropriately used.

In one embodiment, the variant polypeptide may be one in which the amino acid corresponding to position 272 of SEQ ID NO: 1 is substituted with any one selected from the group consisting of glycine, alanine, arginine, valine, leucine, methionine, threonine, asparagine, glutamine, proline, serine, tryptophan, phenylalanine, histidine, cysteine, tyrosine, lysine, aspartate, and glutamic acid.

In any one of the above-described embodiments, the variant polypeptide provided herein may be one in which the amino acid corresponding to position 272 from the N-terminus of SEQ ID NO: 1 is substituted with an amino acid having a polar or hydrophilic side chain (polar amino acid), i.e., an amino acid selected from serine, threonine, cysteine, tyrosine, asparagine, and glutamine. Specifically, the amino acid may be an amino acid selected from threonine, tyrosine, asparagine, and glutamine.

In any one of the above-described embodiments, the variant polypeptide provided herein may be one in which the amino acid corresponding to position 272 from the N-terminus of SEQ ID NO: 1 is substituted with an amino acid having an uncharged side chain (referred to as uncharged amino acid; neutral amino), i.e., an amino acid selected from glycine, alanine, valine, leucine, isoleucine, methionine, phenylalanine, tryptophan, proline, serine, threonine, cysteine, tyrosine, asparagine, and glutamine. Specifically, the amino acid may be an amino acid selected from valine, threonine, tyrosine, asparagine, and glutamine.

In any one of the above-described embodiments, the variant polypeptide having propionaldehyde dehydrogenase activity of the present disclosure may be a polypeptide in which the amino acid corresponding to position 272 of SEQ **ID** NO: 1 is substituted from isoleucine to an amino acid selected from valine, threonine, tyrosine, asparagine, and glutamine, but is not limited thereto.

Meanwhile, those skilled in the art can grasp the amino acid corresponding to position 272 of the amino acid sequence of SEQ **ID** NO: 1 of the present disclosure in any amino acid sequence through sequence alignment known in the art, and even if not separately described herein, it is apparent that the phrase "an amino acid at a particular position in a particular sequence number" obviously includes even "an amino acid at a position corresponding" thereto in any amino acid sequence.

In one embodiment, the variant polypeptide having propionaldehyde dehydrogenase activity of the present disclosure may have a sequence homology of 60% or more and less than 100% to the amino acid sequence of SEQ **ID** NO: 1, specifically a sequence homology of 80% or more and less than 100%, but is not limited thereto.

Specifically, the variant polypeptide of the present disclosure may include one in which the amino acid at position 272 from the N-terminus of SEQ **ID** NO: 1 is substituted with another amino acid in an amino acid sequence having at least 60%, 70%, 75%, 80%, 85%, 90%, 95%, 96%, 97%, 98%, 99%, 99.5%, 99.7% or 99.9% or more homology or identity to the amino acid sequence described by SEQ ID NO: 1. Additionally, it is apparent that any variant having an amino acid sequence, in which part of the sequence is deleted, modified, substituted, conservatively substituted or added, may also fall within the scope of the present disclosure as long as the amino acid sequence has such a homology or identity, and exhibits an efficacy corresponding to the variant of the present disclosure.

In another embodiment, the variant polypeptide may have and/or include an amino acid sequence selected from the group consisting of SEQ ID NOS: 3 to 7 and SEQ ID NOS: 33 to 46, or may essentially consist of the above amino acid sequence. Additionally, the variant polypeptide may include an amino acid sequence having at least 60%, 70%, 75%, 80%, 85%, 90%, 95%, 96%, 97%, 98%, 99%, 99.5%, 99.7% or 99.9% or more homology or identity to the amino acid sequence selected from the group consisting of the amino acid sequence of SEQ ID NOS: 3 to 7 and SEQ ID NOS: 33 to 46.

For example, it may be a case of sequence additions or deletions that do not alter the function of the variant of the present disclosure, naturally occurring mutations, silent mutations thereof, or conservative substitutions at the N-terminus, C-terminus and/or within the amino acid sequences.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue.

For example, positively charged (basic) amino acids include arginine, lysine, and histidine; negatively charged (acidic) amino acids include glutamic acid and aspartic acid; aromatic amino acids include phenylalanine, tryptophan, and tyrosine; and hydrophobic amino acids include alanine, valine, isoleucine, leucine, methionine, phenylalanine, tyrosine, and tryptophan. Additionally, amino acids can be classified into amino acids with electrically charged side chains and amino acids with uncharged side chains, and the amino acids with electrically charged side chains include aspartic acid, glutamic acid, lysine, arginine, and histidine, and the amino acids with uncharged side chains can be further classified into nonpolar amino acids or polar amino acids. The non-polar amino acids include glycine, alanine, valine, leucine, isoleucine, methionine, phenylalanine, tryptophan, and proline; and the polar amino acids include serine, threonine, cysteine, tyrosine, asparagine, and glutamine. Typically, conservative substitutions may have little or no effect on the activity of a protein or polypeptide.

In one embodiment, the variant polypeptide having propionaldehyde dehydrogenase activity of the present disclosure may have an enhanced propionaldehyde dehydrogenase activity, but is not limited thereto. Additionally, the variant of the present disclosure may have activity of increasing the production ability of 3HP-CoA or derivatives thereof, as compared to a wild-type polypeptide having propionaldehyde dehydrogenase activity, but is not limited thereto.

Another aspect of the present disclosure provides a polynucleotide encoding the variant polypeptide.

As used herein, the term "polynucleotide", which is a polymer of nucleotides composed of nucleotide monomers connected in a lengthy chain by a covalent bond, is a DNA or RNA strand having at least a certain length. More specifically, it may mean a polynucleotide fragment encoding the variant.

The polynucleotide encoding the variant polypeptide having propionaldehyde dehydrogenase activity of the present disclosure may include any polynucleotide sequence encoding the variant polypeptide having propionaldehyde dehydrogenase activity of the present disclosure without limitation. For example, the polynucleotide encoding the variant polypeptide having propionaldehyde dehydrogenase activity of the present disclosure may be a polynucleotide sequence encoding the amino acid sequence of the variant polypeptide having propionaldehyde dehydrogenase activity of the present disclosure, but is not limited thereto.

The polynucleotide of the present disclosure may undergo various modifications in the coding region within the scope that does not change the amino acid sequence of the variant of the present disclosure, due to codon degeneracy or in consideration of the codons preferred in an organism in which the variant of the present disclosure is to be expressed. Therefore, it will be apparent that a polynucleotide that may be translated into the polypeptide consisting of the amino acid sequence of the variant of the present disclosure or a polypeptide having a homology or identity thereto by codon degeneracy may also be included.

For example, the polynucleotide of the present disclosure may include those in which the codon encoding isoleucine, the amino acid corresponding to position 272 of SEQ ID NO: 2, is substituted with a codon encoding another amino acid other than isoleucine, e.g., amino acid selected from valine, threonine, tyrosine, asparagine, and glutamine, in the nucleotide sequence having 60%, 70%, 75%, 80%, 85%, 90%, 95%, 96%, 97%, 98%, 99%, 99.5%, 99.7% or 99.9% or more homology or identity with the sequence of SEQ ID NO: 2, but is not limited thereto. Additionally, it is apparent that any variant having a polynucleotide sequence, in which part of the sequence is deleted, modified, substituted, conservatively substituted, or added, may also fall within the scope of the present disclosure as long as the polynucleotide sequence has such a homology or identity and encodes the amino acid sequence of the variant polypeptide having propionaldehyde dehydrogenase activity of the present disclosure.

Additionally, the polynucleotide of the present disclosure may include a probe that may be prepared from a known gene sequence, for example, any sequence which may hybridize with a sequence complementary to all or part of the polynucleotide sequence of the present disclosure under stringent conditions without limitation. The "stringent conditions" refers to conditions under which specific hybridization between polynucleotides is allowed. Such conditions are specifically described in the literature (e.g., Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having a high homology or identity, i.e., polynucleotides having 60% or more, 70% or more, 75% or more, 80% or more, 85% or more, 90% or more, 95% or more, 96% or more, 97% or more, 98% or more, or 99% or more hybridize with each other, while polynucleotides having a homology or identity lower than the above homology or identity do not hybridize with each other; or may include ordinary washing conditions of Southern hybridization, *i.e.*, washing once, specifically twice or three times, at a salt concentration and a temperature corresponding to 60°C., 1×SSC, 0.1% SDS, specifically 60°C., 0.1xSSC, 0.1% SDS, and more specifically 68°C., 0.1×SSC, 0.1% SSD.

Hybridization requires that two nucleic acids have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the polynucleotide of the present disclosure may also include an isolated nucleic acid fragment complementary to the entire sequence as well as a nucleic sequence substantially similar thereto.

Specifically, polynucleotides having a homology or identity to the polynucleotide of the present disclosure may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (e.g., Sambrook et al.).

As used herein, the term "homology" or "identity" refers to the degree of similarity between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codon or degenerate codons in hybridizing polynucleotides is also included.

Whether any two polynucleotide or polypeptide sequences have a homology, similarity, or identity may be, for example, determined by a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277) (preferably, version 5.0.0 or versions thereafter) (GCG program package (Devereux, J., et al., Nucleic Acids Research 12: 387 (1984)), BLASTP, BLASTN, FASTA (Atschul, [S.] [F.,] [ET AL., J MOLEC BIOL 215]: 403 (1990); Guide to Huge Computers, Martin J. Bishop, [ED.,] Academic Press, San Diego, 1994, and [CARILLO ETA/.](1988) SIAM J Applied Math 48: 1073). For example, the homology, similarity, or identity may be determined using BLAST or ClustalW of the National Center for Biotechnology Information (NCBI).

The homology, similarity, or identity of polynucleotides or polypeptides may be, for example, determined by comparing sequence information using, for example, the GAP computer program, such as Needleman et al. (1970), J Mol Biol. 48: 443 as disclosed in Smith and Waterman, Adv. Appl. Math (1981) 2:482. In summary, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps. Therefore, the term "homology" or "identity" used herein indicates relevance between sequences.

Still another aspect of the present disclosure provides a vector containing the polynucleotide of the present disclosure. The vector may be an expression vector for expressing the polynucleotide in a microorganism, but is not limited thereto.

As used herein, the term "vector" may include a DNA construct containing the nucleotide sequence of a polynucleotide encoding a target polypeptide operably linked to a suitable expression regulatory region (expression regulatory sequence) so as to be able to express the target polypeptide in a suitable host cell. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pBR, pUC, pBluescriptII, pGEM, pTZ, pCL and pET, *etc.* may be used. Specifically, pDZ, pDC, pDCM2, pACYC177, pACYC184, pCL, pECCG117, pUC19, pBR322, pMW118, pCC1BAC, pFS45 vector, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or a microorganism so that the polypeptide encoded by the polynucleotide can be expressed in the microorganism. As long as the transformed polynucleotide can be expressed in the microorganism, it does not matter whether the transformed polynucleotide is integrated into the chromosome and located therein, or located extrachromosomally, and both cases can be included. Additionally, the polynucleotide may include DNA and/or RNA encoding the target polypeptide. The polynucleotide may be introduced in any form, as long as it can be introduced into the microorganism and expressed therein. For example, the polynucleotide may be introduced into the microorganism in the form of an expression cassette, which is a gene construct including all elements required for its autonomous expression. The expression cassette may commonly include a promoter operably linked to the polynucleotide, a transcription termination signal, a ribosome-binding site, and a translation termination signal. The expression cassette may be in the form of a self-replicable expression vector. Additionally, the polynucleotide may be introduced into a microorganism as it is and operably linked to sequences required for expression in the microorganism, but is not limited thereto.

Further, as used herein, the term "operably linked" means that the polynucleotide sequence is functionally linked to a promoter sequence that initiates and mediates transcription of the polynucleotide encoding the target variant of the present disclosure.

Still another aspect of the present disclosure provides a microorganism including any one or more of the variant polypeptide, a polynucleotide encoding the variant polypeptide, and a vector containing the polynucleotide.

In one embodiment, the microorganism of the present disclosure may be a microorganism having a production ability of 3HP-CoA or derivatives thereof.

In one embodiment, the microorganism of the present disclosure may be a microorganism having a production ability of a compound selected from the group consisting of 3HP-CoA, 3HP, P3HP, P(3HB-3HP), P(4HB-3HP), P(3HP-3HV), acrylyl-CoA, acrylic acid, and a combination thereof.

As used herein, the term "3-hydroxypropionyl-CoA (3HP-CoA)" is a useful intermediate that can be converted into various substances, such as acrylic acid (AA), 3HP, P3HP, *etc.).*

As used herein, the term 3HP-CoA derivative" refers to a compound that can be obtained by conversion from 3HP-CoA. For example, examples may include 3HP, polyhydroxyalkanoate (PHA) homopolymer, PHA copolymer, PHA homopolymer or copolymer containing 3HP as a monomer, acrylyl-CoA, acrylic acid, *etc.,* but are not limited thereto, and conversion from 3HP-CoA to 3HP-CoA derivatives (various 3HP-CoA) can be easily carried out by methods widely known in the art.

In one specific example, the 3HP-CoA derivatives can be 3HP, P3HP, P(3HB-3HP), P(4HB-3HP), P(3HP-3HV), acrylyl-CoA or acrylic acid, but are not limited thereto.

As used herein, the term "3-hydroxypropionic acid (3HP)", which is a compound having a molecular weight of 90.078 and a chemical formula of C₃H₆O₃, is used as a platform material for the synthesis of commercially important compounds, and specifically used to produce acrylic acid, acrylic ester, methyl acrylate, acrylamide, ethyl 3-hydroxypropionic acid, malonic acid, propiolactone, acrylonitrile, 1,3-propanediol, *etc.* Additionally, 3-hydroxypropionic acid is used in the synthesis of biodegradable polymers as one of the monomer units incorporated into polyhydroxyalkanoates (PHA), and can further be effectively used in the fields of superabsorbent polymers, plastics, and paints, *etc.*

As used herein, the term "PHA homopolymer" is a compound having all identical monomer units. Examples of PHA homopolymers may include poly-3-hydroxyalkanoates, such as poly-3-hydroxypropionic acid (P3HP), poly-3-hydroxybutyrate (PHB), and poly-3-hydroxyvalerate; poly-4-hydroxyalkanoates, such as poly-4-hydroxybutyrate (P4HB), and poly-4-hydroxyvalerate (P4HV); and poly-5-hydroxyalkanoates, such as poly-5-hydroxyvalerate (P5HV), *etc.*

As used herein, the term "poly-3-hydroxypropionic acid (P3HP)" is one of the polyhydroxyalkanoate (PHA) homopolymers, and refers to a compound in which all monomer units are 3-hydroxypropionic acid.

As used herein, the term "PHA copolymer" is a copolymer (containing two or more different monomer units) in which different monomers are randomly distributed in the polymer chain. Examples of PHA copolymers include poly-3-hydroxybutyrate-co-3-hydroxypropionate (P(3HB-3HP)), poly-4-hydroxybutyrate-co-3-hydroxypropionate (P(4HB-3HP)), poly-3-hydroxypropionate-co-3-hydroxyvalerate (P(3HP-3HV)), poly-3-hydroxypropionate-co-3-hydroxyhexanoate (P(3HB-3HH)), poly-3-hydroxybutyrate-co-4-hydroxybutyrate (P(3HB-4HB)), poly-3-hydroxybutyrate-co-4-hydroxyvalerate (P(3HB-4HV)), poly-3-hydroxybutyrate-co-3-hydroxyvalerate (P(3HB-3HV)), poly-3-hydroxybutyrate-co-3-hydroxyhexanoate (P(3HB-3HH)) and poly-3-hydroxybutyrate-co-5-hydroxyvalerate (P(3HB-5HV), *etc.*

As used herein, the term "microorganism (or strain)" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.,* and may be a microorganism including genetic modification to produce a desired polypeptide, protein or product. As used herein, the terms "strain" and "microorganism" have the same meaning and can be interchangeably used without limitation.

As used herein, the term "microorganism for producing 3HP-CoA or derivatives thereof", which is a prokaryotic or eukaryotic microbial strain capable of producing 3HP-CoA or derivatives thereof in an organism, may include both a microorganism, in which a production ability of 3HP-CoA or derivatives thereof has been imparted to a parent strain having no production ability of 3HP-CoA or derivatives thereof, or a microorganism that endogenously has a production ability of 3HP-CoA or derivatives thereof. The production ability of 3HP-CoA or derivatives thereof may be imparted or enhanced by improvement of species.

In one embodiment, the microorganism of the present disclosure may be a microorganism naturally having a production ability of 3HP-CoA or derivatives thereof; or a microorganism in which the variant of the present disclosure or a polynucleotide encoding the same (or a vector containing the polynucleotide) has been introduced and/or a production ability of 3HP-CoA or derivatives thereof has been imparted to a parent strain not having the variant polypeptide having propionaldehyde dehydrogenase activity or a production ability of 3HP-CoA or derivatives thereof, but is not limited thereto.

In one embodiment, the microorganism of the present disclosure may include both a microorganism, which includes the variant polypeptide sequence having propionaldehyde dehydrogenase activity of the present disclosure due to the mutation of a gene encoding the variant polypeptide having propionaldehyde dehydrogenase activity of the present disclosure on the chromosome, and/or a microorganism, which includes the variant polypeptide having propionaldehyde dehydrogenase activity of the present disclosure by introducing a vector containing a polynucleotide encoding the variant polypeptide having propionaldehyde dehydrogenase activity of the present disclosure, but is not limited thereto.

As used herein, the term "non-modified microorganism" does not exclude a strain containing a mutation that may occur naturally in a microorganism, and may refer to a wild-type strain or natural-type strain itself, or a strain before its trait is altered due to genetic modification caused by natural or artificial factors. For example, the non-modified microorganism" may refer to a strain into which the variant polypeptide having propionaldehyde dehydrogenase activity described herein is not introduced, or a strain before the introduction thereof. The "non-modified microorganism" may be used interchangeably with "strain before modification", "microorganism before modification", "non-mutant strain", "non-modified strain", "non-mutant microorganism", or "reference microorganism.

The microorganism having the production ability of 3HP-CoA or derivatives thereof of the present disclosure may be a microorganism including any one or more of the variant of the present disclosure, the polynucleotide of the present disclosure, and a vector containing the polynucleotide of the present disclosure; a microorganism modified to express the variant of the present disclosure or the polynucleotide of the present disclosure; a microorganism expressing the variant of the present disclosure or the polynucleotide of the present disclosure (*e.g*., a recombinant strain); or a microorganism having the variant activity of the present disclosure (*e.g.*, a recombinant strain), but is not limited thereto.

In one example, the strain of the present disclosure, which is a cell or microorganism transformed with the polynucleotide of the present disclosure or a vector containing the polynucleotide encoding the variant of the present disclosure to express the variant of the present disclosure, may include all microorganisms capable of producing 3HP-CoA or derivatives thereof, including the variant of the present disclosure. For example, the microorganism of the present disclosure may be a recombinant strain in which the production ability of 3HP-CoA or derivatives thereof is increased by introducing the polynucleotide encoding the variant of the present disclosure into a natural wild-type microorganism or a microorganism having a production ability of 3HP-CoA or derivatives thereof, thereby expressing the variant polypeptide. The recombinant strain having an increased production ability of 3HP-CoA or derivatives thereof may be a microorganism having an increased production ability of 3HP-CoA or derivatives thereof, as compared to a natural wild-type microorganism or a non-modified microorganism of propionaldehyde dehydrogenase (e.g., a microorganism expressing a wild-type propionaldehyde dehydrogenase or a microorganism not expressing the variant of the present disclosure), but is not limited thereto. In one example, the microorganism with an increased production ability of 3HP-CoA or derivatives thereof may be a microorganism having an increased production ability of 3HP-CoA or derivatives thereof, as compared to a microorganism including the polypeptide of SEQ ID NO: 1 or a polynucleotide encoding the same, but is not limited thereto. In one example, the non-modified microorganism, which is the target strain for comparing the increase in the production ability of 3HP-CoA or derivatives thereof, may be the MBX184 strain (US 6329183 B1), but is not limited thereto.

The microorganism of the present disclosure may include all microorganisms capable of expressing the variant polypeptide having propionaldehyde dehydrogenase activity of the present disclosure by various known methods in addition to the introduction of the nucleic acid or vector.

In one example, the microorganism having an increased production ability of 3HP-CoA or derivatives thereof may have an increased production ability of 3HP-CoA or derivatives thereof by about 1% or more, specifically about 1% or more, about 2% or more, about 3% or more, about 5% or more, about 10% or more, about 15% or more, about 20% or more, about 25% or more, about 30% or more, about 35% or more, about 40% or more, about 45% or more, about 50% or more, about 51% or more, about 52% or more, about 53% or more, about 54% or more, or about 55% or more (the upper limit is not particularly limited, for example, about 200% or less, about 150% or less, about 100% or less, about 90% or less, about 80% or less, about 70% or less, about 65% or less, or about 60% or less) as compared to the production ability of 3HP-CoA or derivatives thereof of a parent strain before modification or a non-modified microorganism, but is not limited thereto, as long as it has an increased + value compared to the production ability of a parent strain before modification or a non-modified microorganism. In another example, the recombinant strain having an increased production ability of 3HP-CoA or derivatives thereof may have an increased production ability of 3HP-CoA or derivatives thereof by about 1.01 times or more, about 1.02 times or more, about 1.03 times or more, about 1.05 times or more, about 1.1 times or more, about 1.15 times or more, about 1.20 times or more, about 1.25 times or more, about 1.30 times or more, about 1.35 times or more, about 1.40 times or more, about 1.45 times or more, about 1.50 times or more, or about 1.55 times or more (the upper limit is not particularly limited, for example, about 10 times or less, about 5 times or less, about 3 times or less, about 2 times or less, or about 1.6 times or less) as compared to that of a parent strain before modification or a non-modified microorganism, but is not limited thereto. As used herein, the term "about" refers to a range which includes all of ±0.5, ±0.4, ±0.3, ±0.2, ±0.1, *etc.,* and includes all of the values equivalent to those which come immediately after the term "about" or those in a similar range, but the range is not limited thereto.

The microorganism of the present disclosure may be a microorganism belonging to *Escherichia sp., Erwinia sp., Serratia sp., Providencia sp., Corynebacteria sp., Pseudomonas sp., Leptospira sp., Salmonella sp., Brevibacteria sp., Hypomononas sp., Chromobacterium sp.,* and *Norcardia sp.,* or fungi or yeasts, specifically, a microorganism belonging to *Escherichia sp.,* more specifically, *Escherichia coli* (*E. coli*)*,* but is not limited thereto.

Meanwhile, the microorganism belonging to *Escherichia sp.* having a production ability of 3HP-CoA or derivatives thereof of the present disclosure may include all of the following: a natural wild-type microorganism itself; a microorganism belonging to *Escherichia sp.* in which the activity of a gene involved in the production mechanism of 3HP-CoA or derivatives thereof is enhanced or weakened so as to have an improved production ability of 3HP-CoA or derivatives thereof; or a microorganism belonging to *Escherichia sp.* in which the activity of a foreign gene is introduced or enhanced so as to have an improved production ability of 3HP-CoA or derivatives thereof.

In one embodiment, the microorganism belonging to *Escherichia sp.* having a production ability of 3HP-CoA or derivatives thereof of the present disclosure may be one in which a gene involved in P3HP production mechanism, specifically, glycerol dehydratase and PHA synthase, more specifically, glycerol dehydratase derived from *Klebsiella pneumoniae* and PHA synthase derived from *Ralstonia eutropha,* is introduced, but is not limited thereto. In another embodiment, the microorganism belonging to *Escherichia sp.* having a production ability of 3HP-CoA or derivatives thereof of the present disclosure may be one in which a gene related to the mechanism of using 1,3-propanediol as a carbon source or producing 1,3-propanediol, specifically, 1,3-propanediol dehydrogenase, alcohol dehydrogenase, or aldehyde reductase, more specifically, 1,3-propanediol dehydrogenase derived from *Klebsiella pneumoniae* may be introduced, or the activity of alcohol dehydrogenase or aldehyde reductase derived from *Escherichia coli* is enhanced, but is not limited thereto. US 6329183 B1 is incorporated herein by reference in its entirety.

Additionally, the microorganism of the present disclosure may be one in which the activity of propionaldehyde dehydrogenase is enhanced as compared to the parent strain.

As used herein, the term "enhancement" of polypeptide activity means that the activity of a polypeptide is increased, as compared with its endogenous activity. The increase may be used interchangeably with terms such as activation, up-regulation, overexpression, increase, *etc.* In particular, the activation, enhancement, up-regulation, overexpression and increase may include both cases in which an activity not originally possessed is exhibited, or an activity is enhanced compared to the endogenous activity or the activity before modification. The "endogenous activity" refers to the activity of a particular polypeptide originally possessed by a parent strain before transformation or a non-modified microorganism when a trait is altered by genetic variation due to a natural or artificial factor. The endogenous activity may be interchangeably used with "activity before modification".

The "enhancement", "up-regulation", "overexpression", or "increase" in the activity of a polypeptide, as compared with its endogenous activity, means that the activity of a polypeptide is enhanced, as compared with the activity and/or concentration (expression level) of a particular polypeptide originally possessed by a parent strain before transformation or a non-modified microorganism.

The enhancement of the activity of the polypeptide may be carried out by various methods well known in the art. Examples of the methods may include a method of increasing the intracellular copy number of a gene encoding the variant, a method of introducing a mutation in the expression regulatory sequence of a gene encoding the variant on the chromosome, a method of substituting the expression regulatory sequence of a gene encoding the variant on the chromosome with a sequence having strong activity, a method of substituting the gene encoding the variant on the chromosome with a mutated gene to increase the activity of the variant, and a method of introducing a mutation in the gene encoding the variant protein on the chromosome to enhance the activity of the variant, but are not limited thereto.

As used herein, the term "introduction" refers to a method of delivering a polynucleotide encoding the propionaldehyde dehydrogenase or a vector containing the same to a host cell. Such introduction may be easily performed by a common method in the art. In general, examples of the method include a CaCl₂ precipitation method, a Hanahan method with improved efficiency using dimethyl sulfoxide (DMSO) as a reducing agent in the CaCl₂ precipitation method, an electroporation method, a calcium phosphate precipitation method, a protoplast fusion method, a stirring method using silicon carbide fibers, a transformation method using PEG, a dextran sulfate-, lipofectamine-, and dry/suppression-mediated transformation method, *etc.* The method for transforming the vector is not limited to the above exemplified methods, and any transformation or transfection method commonly used in the art may be used without limitation. The delivered polynucleotide may be inserted into the chromosome of a microorganism and located therein or located extrachromosomally, as long as the polynucleotide can be expressed in the host cell. Alternatively, the polynucleotide may be introduced in any form as long as it can be introduced into the host cell and expressed therein. For example, the polynucleotide may be introduced into a host cell in the form of an expression cassette, which is a polynucleotide construct including all the essential elements required for its self-expression, but is not limited thereto. The expression cassette conventionally includes a promoter operably linked to the open reading frame (hereinafter, "ORF") of the gene, a transcription termination signal, a ribosome-binding site, and a translation termination signal. The expression cassette may be in the form of a self-replicable expression vector. Additionally, the polynucleotide may be introduced in the form as it is into a host cell and operably linked to a sequence required for its expression in the host cell, but is not particularly limited thereto.

Still another aspect of the present disclosure provides a method for producing 3HP-CoA or derivatives thereof, including culturing the microorganism of the present disclosure in a medium.

Specifically, the method for producing 3HP-CoA or derivatives thereof of the present disclosure may include culturing a microorganism, which includes any one or more of the variant of the present disclosure, the polynucleotide of the present disclosure, and the vector of the present disclosure, in a medium, but is not limited thereto.

As used herein, the term "cultivation" means that the microorganism of the present disclosure is grown under appropriately controlled environmental conditions. The cultivation process of the present disclosure may be performed in a suitable culture medium and culture conditions known in the art. Such a cultivation process may be easily adjusted for use by those skilled in the art according to the microorganism to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of the microorganism of the present disclosure as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, *etc.,* while adjusting temperature, pH, *etc.*

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* and amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e.,* molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors, *etc.,* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected to the medium in order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature during the cultivation of the present disclosure may be in the range from 20°C to 45°C, specifically from 25°C to 40°C, and the cultivation may be continued for about 10 hours to 160 hours, but is not limited thereto.

The 3HP-CoA or derivatives thereof produced by the cultivation of the present disclosure may be released into the medium or remain in the cells.

In one embodiment, the method for producing 3HP-CoA or derivatives thereof of the present disclosure may further include a step of preparing the microorganism of the present disclosure, a step of preparing a medium for culturing the microorganism, or a combination thereof (regardless of the order, in any order), for example, prior to the culturing step.

In one embodiment, the method for producing 3HP-CoA or derivatives thereof of the present disclosure may further include a step of recovering 3HP-CoA or derivatives thereof from the cultured medium (medium on which the cultivation was performed), or from the microorganism of the present disclosure. The recovering step may be further included after the culturing step.

In the recovering step, 3HP-CoA or derivatives thereof may be collected using the method of culturing the microorganism of the present disclosure, for example, using a suitable method known in the art according to a batch culture, continuous culture, or fed-batch culture method, *etc.* For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC, or a combination thereof may be used, and 3HP-CoA or derivatives thereof can be recovered from the medium or microorganism using suitable methods known in the art.

Additionally, the method for producing 3HP-CoA or derivatives thereof of the present disclosure may further include a purification step, which may be performed using an appropriate method known in the art. In one example, when the method for producing 3HP-CoA or derivatives thereof of the present disclosure includes both a recovering step and a purification step, the recovering step and the purification step may be performed continuously or intermittently regardless of the order, or simultaneously, or may be integrated into one step, but the method is not limited thereto.

Still another aspect of the present disclosure provides a composition for producing 3HP-CoA or derivatives thereof, including the variant polypeptide of the present disclosure; a polypeptide encoding the variant polypeptide; a vector containing the polynucleotide; a microorganism including any one or more of the variant polypeptide of the present disclosure, a polynucleotide encoding the variant polypeptide, and a vector containing the polynucleotide; a culture product of the microorganism; or two or more thereof.

The composition of the present disclosure may further include any suitable excipient commonly used in compositions for producing 3HP-CoA or derivatives thereof, and such excipients may include, for example, preservatives, wetting agents, dispersing agents, suspending agents, buffers, stabilizers, or isotonic agents, *etc.,* but are not limited thereto.

In one embodiment, each component present in the composition of the present disclosure may be contained in a microbiologically effective amount, or in an amount that can be appropriately present in the composition for production.

Still another aspect of the present disclosure provides use of the variant polypeptide of the present disclosure or the microorganism of the present disclosure for the production of 3HP-CoA or derivatives thereof.

### [Mode for Carrying Out the Invention]

Hereinafter, the present disclosure will be described in detail by way of Examples. However, these Examples are merely preferred Examples given for illustrative purposes, and thus, the scope of the invention is not intended to be limited to or by these Examples. Meanwhile, technical features which are not described herein can be sufficiently understood and easily carried out by those skilled in the art in the technical field of the present disclosure or in a similar technical field.

### Example 1: Construction of DNA Library Encoding Mutated Propionaldehyde Dehydrogenase using Artificial Mutagenesis

In this Example, a vector library for obtaining propionaldehyde dehydrogenase variants was prepared by the following method. Error-prone PCR was performed for the *pduP* gene (SEQ ID NO: 2) encoding propionaldehyde dehydrogenase (SEQ ID NO: 1) derived from *Salmonella enterica,* and thereby *pduP* gene mutants (1,395bp) randomly introduced with a mutation of nucleotide substitution were obtained. The error-prone PCR was performed by the GenemorphII Random Mutagenesis Kit (Agilent Technologies, Inc., Santa Clara, CA, USA), using the synthesized wild-type *pduP* gene as a template along with primer 1 (SEQ ID NO: 8) and primer 2 (SEQ ID NO: 9) as shown in Table 1.

**[Table 1]**

| Primer | Nucleotide Sequence |
|---|---|
| SEQ ID NO: 8 | 5'- CTAGGATTCAGGAGGTTTTT ATGAATACTTCTGAACTCGAAACC -3' |
| SEQ ID NO: 9 | 5'- CTCAAAAGGGTCGACAAATC TTAGCGAATAGAAAAGCCGTTGGTC -3' |

The amplified gene fragments were introduced with 0 to 2 mutations per 1 kb, and PCR was performed for a total of 30 cycles under the following conditions: 95°C denaturation for 30 seconds, annealing at 55°C for 30 seconds, and polymerization at 72°C for 1 minute.

It was attempted to introduce the amplified fragments so as to be expressed as one operon under the control of the trc promoter together with glycerol dehydratase derived from *Klebsiella pneumoniae* and PHA synthase derived from *Ralstonia eutropha* with reference to the pFS45 plasmid (US 6329183 B1), and accordingly the above fragments were linked to fragments, which were amplified by PCR with primer 3 (SEQ ID NO: 10) and primer 4 (SEQ ID NO: 11) as shown in Table 2 using the pFS45 plasmid as a template, by In-Fusion HD Cloning Kit(Takara Bio Inc., Kusatsu, Japan), and then transformed into *E*. *coli* DH5α. The pFS45 plasmid used as a template contains the *lacl* gene derived from pSE380 (Thermo Fisher Scientific Inc., Waltham, MA, USA), the ampicillin resistance gene (*ampR*)*,* and the origin of replication (Ori).

**[Table 2]**

| Primer | Nucleotide Sequence |
|---|---|
| SEQ ID NO: 10 | 5'- GATTTGTCGACCCTTTTGAGCCGATG -3' |
| SEQ ID NO: 11 | 5'- AAAAACCTCCTGAATCCTAGAGGATC -3' |

After transformation, the transformants were plated on a solid LB medium containing ampicillin (100 mg/L). 20 transformed colonies were selected and then plasmids were obtained therefrom. Upon analysis of the nucleotide sequences of the plasmids, it was confirmed that mutations were introduced to different positions with a frequency of 0.9 mutations/kb. About 10,000 transformed *E*. *coli* colonies were collected and their plasmids were extracted, which were named *"pFS-pduP(mt)"* library.

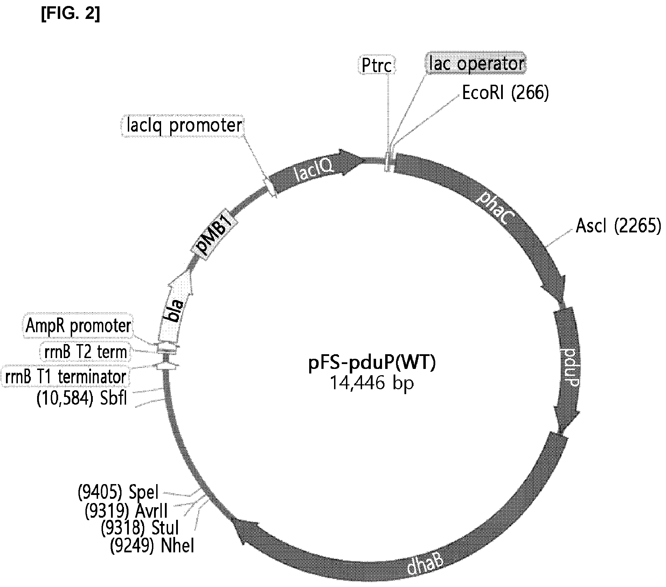

Additionally, a plasmid having the wild-type *pduP* gene to be used as a control was prepared. PCR was performed using the synthesized wild-type *pduP* gene as a template along with primer 1 (SEQ ID NO: 8) and primer 2 (SEQ ID NO: 9), under the same conditions described above, and a plasmid was prepared in the same form. The thus-prepared plasmid was named pFS-pduP(WT), and its map is shown in FIG. 2.

### Example 2: Preparation of Strain Library of Propionaldehyde Dehydrogenase Variants and Selection of Strains with Increased Production Ability of P3HP

In this Example, the *pFS-pduP(mt)* library prepared in Example 1 was transformed by homologous recombination using the MBX184 strain (US 6593116 B1), which is the wild-type *E*. *coli LS5218* strain, as a parent strain, in order to prepare a strain library for variants, and the transformant was plated on a solid LB medium containing ampicillin (100 mg/L) and about 5,000 colonies were obtained therefrom. The colonies were named from *184*/*pFS-pduP(mt)-1* to *184*/*pFS-pduP(mt)-5000.* In addition, the pFS-pduP(WT) vector prepared in Example 1 was transformed to prepare a control strain, and it was named *184*/*pFS-pduP(WT).*

About 5,000 colonies obtained above were each inoculated into a liquid LB medium (350 µL) containing ampicillin (100 mg/L) and cultured in a 96-well plate at 37°C at a rate of 1,000 rpm for 16 hours. The liquid LB medium was prepared with LB broth (Merck, Darmstadt, Germany) at 25 g/L to be used. Thereafter, each culture was centrifuged (2,000Xg, 10 min), the supernatant was removed, and resuspended in 350 µL of a selective medium. The selective medium was prepared to contain 2.5 g of LB broth powder, 50 mmol of phosphate buffer (pH 7), 10 g of glycerol, 2 g of glucose, 5 nmol of coenzyme B-12, 100 µg of ampicillin, and 0.1 mmol of isopropyl β-D-1-thiogalactopyranoside (IPTG) per 1 L, and then dispensed in a 96-well plate to be used. After culturing the resuspended culture at 30°C at a rate of 1,000 rpm for about 24 hours, the strains expected to have an increased production ability of P3HP were selected based on the absorbance measured at a wavelength of 600 nm. This is based on the finding that when propionaldehyde dehydrogenase is weakened, its substrate, 3-hydroxypropionaldehyde (3HPA), accumulates and the growth of the microorganism is inhibited, and an increase in P3HP production leads to an increase in the cell absorbance (OD) due to P3HP granules stored within the cell. The variant strains showing an absorbance increased by more than 20% were selected as compared to the control strain. As shown in Table 3, among the selected variant strains, 184/pFS-pduP(mt)-2101 showing an absorbance increased by 26.5% compared to the control 184/pFS-pduP(WT) was selected. Other colonies showed similar or reduced absorbance compared to the control strain.

**[Table 3]**

| | Strain | OD(600 nm) |
|---|---|---|
| Control | 184/pFS-pduP(WT) | 1.85 |
| Selected Experimental Group | 184/pFS-pduP(mt)-2101 | 2.34 |

### Example 3: Confirmation of P3HP Production Ability of Strains Selected from the Strain Library of Propionaldehyde Dehydrogenase Variants and Confirmation of pduP Gene Mutation

To confirm whether the P3HP production ability of the 184/pFS-pduP(mt)-2101 strain obtained in Example 2 was improved compared to the control 184/pFS-pduP(WT) strain, the two strains were each inoculated into 100 mL of a liquid LB medium containing ampicillin (100 mg/L) and cultured in a 500 mL-baffle flask at 37°C at a rate of 225 rpm for 16 hours. The liquid LB medium was prepared with LB broth (Merck, Darmstadt, Germany) at 25 g/L to be used. Thereafter, each culture was centrifuged (2,000Xg, 10 min), the supernatant was removed, and resuspended in 100 mL of a production medium. The production medium was prepared to contain 2.5 g of LB broth powder, 50 mmol of phosphate buffer (pH 7), 10 g of glycerol, 2 g of glucose, 5 nmol of coenzyme B-12, 100 µg of ampicillin, and 0.1 mmol of isopropyl β-D-1-thiogalactopyranoside (IPTG) per 1 L, and then dispensed in a 500 mL-baffle flask to be used.

The resuspended culture was cultured at 30°C at a rate of 225 rpm for about 48 hours, the absorbance was measured at a wavelength of 600 nm, and 30 mL of each culture was centrifuged to remove the supernatant.

The recovered cells were washed once with distilled water, lyophilized, and added to 2 mL of butanolysis solvent to be used for the butanolysis reaction (110°C, 3 hours). The butanolysis solvent was prepared by mixing N-butanol and concentrated hydrogen chloride solution in a volume ratio of 9:1, and benzoic acid was added at 2 mg/mL as an internal standard. The water-soluble components were removed by layer separation by adding 3 mL of distilled water to the solution after completion of the reaction, and the organic solvent layer was used for GC (gas chromatography) analysis. 1 µL of sample was used at a fraction ratio of 1:50 and a flow rate of 2 mL/min. As the GC column, the SPB-1 fused silica capillary GC column (30 m, 0.32 mm ID, 0.25 µm film; Merck, Darmstadt, Germany) was used, and the temperature was set to 80°C for 2 minutes, and then increased by 10°C per min to reach 250°C, and maintained at 250°C for 2 minutes. β-Propiolactone was used as a standard material for quantifying P3HP.

As a result of the analysis, as shown in Table 4 below, the amount of P3HP produced using the selected strain 184/pFS-pduP(mt)-2101 increased by 47% and its titer by 67%, as compared to the control strain 184/pFS-pduP(WT).

**[Table 4]**

| | Strain | OD(600 nm) | DCW(g/L) | P3HP Productio n (%) | P3HP Titer (mg/L) |
|---|---|---|---|---|---|
| Control | 184/pFS-pduP(WT) | 2.91 | 0.79 | 6.05 | 48.6 |
| Selected Experimental Group | 184/pFS-pduP(mt)-2101 | 3.33 | 0.91 | 8.87 | 81.2 |

Subsequently, to confirm random mutations introduced into the propionaldehyde dehydrogenase of the one selected strain, the nucleotide sequences of the *pduP* gene were analyzed. For analyzing the nucleotide sequences, primer 5 (SEQ ID NO: 12) and primer 6 (SEQ ID NO: 13) were used as shown in Table 5 below.

**[Table 5]**

| Primer | Nucleotide Sequence |
|---|---|
| SEQ ID NO: 12 | 5'- ATATCGCCGGTGTGATCAACCC -3' |
| SEQ ID NO: 13 | 5'- TGCGCTCAACGTTGATCGCGTAATCGGCG -3' |

After analyzing the nucleotide sequences of the obtained mutant *pduP* gene, the nucleotide sequences of the mutant *pduP* gene were confirmed by comparing with the nucleotide sequences of the wild-type *pduP* gene of SEQ ID NO: 2, and based on this, the amino acid sequences of the variant propionaldehyde dehydrogenase were confirmed. As shown in Table 6 below, it was confirmed that the amino acid at position 272 of the propionaldehyde dehydrogenase variant of the selected 184/pFS-pduP(mt)-2101 strain was mutated from isoleucine to threonine.

**[Table 6]**

| Strain | Amino acid mutation of propionaldehyde dehydrogenase |
|---|---|
| 184/pFS-pduP(mt)- 2101 | I272T |

### Example 4: Preparation of Vectors Containing DNA Encoding Propionaldehyde Dehydrogenase with Different Amino Acids Substituted at Same Mutation Position and Comparison of P3HP Production Ability Thereof

To confirm the effect the mutation position in the propionaldehyde dehydrogenase variant identified in Example 3 above, vectors containing avariant, in which the amino acid at position 272 was substituted with an amino acid other than isoleucine or threonine, were prepared.

To prepare the vectors, first, PCR was performed using the synthesized wild-type *pduP* gene as a template along with primer 1 (SEQ ID NO: 8), and primer 7 (SEQ ID NO: 14), primer 8 (SEQ ID NO: 15) as shown in Table 7 below, and primer 2 (SEQ ID NO: 9) to amplify a DNA fragment of about 830 bp and a DNA fragment of about 600 bp for each. PCR was performed for a total of 30 cycles under the following conditions: 95°C denaturation for 30 seconds, annealing at 55°C for 30 seconds, and polymerization at 72°C for 30 seconds.

**[Table 7]**

| Primer | Nucleotide Sequence |
|---|---|
| SEQ ID NO: 14 | 5'- GCAGGGCAGGTTGTAATCGAATGAC -3' |
| SEQ ID NO: 15 | 5'- TCGATTACAACCTGCCCTGCggtGCCGAGAAGAGCCTGATCGTAG - 3' |

In the same manner as Example 1 above, the amplified gene fragments were linked to the pFS45 plasmid, which was amplified by PCR with primer 3 (SEQ ID NO: 10) and primer 4 (SEQ ID NO: 11), by In-Fusion HD Cloning Kit (Takara Bio Inc., Kusatsu, Japan), and then transformed into *E*. *coli* DH5α to obtain a plasmid vector containing a mutation, in which the amino acid at position 272 of propionaldehyde dehydrogenase was substituted with glycine, and the prepared plasmid was named pFS-pduP(I272G).

In the same manner, 17 plasmids containing mutations, in which the amino acid at position 272 of propionaldehyde dehydrogenase was substituted with an amino acid other than isoleucine, threonine, or glycine, were additionally prepared using primers containing the nucleotide sequences encoding the amino acids to be substituted. The primer sequences used are as shown in Table 8 below, and the amino acids to be substituted are indicated.

**[Table 8]**

| SEQ ID NO: | Sequences | Substituted Amino Acids |
|---|---|---|
| 15 | 5'-TCGATTACAACCTGCCCTGCggtGCCGAGAAGAGCCTGATCGTAG-3' | Glycine |
| 16 | 5'-TCGATTACAACCTGCCCTGCgcgGCCGAGAAGAGCCTGATCGTAG- 3' | Alanine |
| 17 | 5'-TCGATTACAACCTGCCCTGCgtgGCCGAGAAGAGCCTGATCGTAG-3' | Valine |
| 18 | 5'-TCGATTACAACCTGCCCTGCctgGCCGAGAAGAGCCTGATCGTAG-3' | Leucine |
| 19 | 5'-TCGATTACAACCTGCCCTGCatgGCCGAGAAGAGCCTGATCGTAG-3' | Methionine |
| 20 | 5'-TCGATTACAACCTGCCCTGCtttGCCGAGAAGAGCCTGATCGTAG-3' | Phenylalanine |
| 21 | 5'-TCGATTACAACCTGCCCTGCtggGCCGAGAAGAGCCTGATCGTAG-3' | Tryptophan |
| 22 | 5'-TCGATTACAACCTGCCCTGCccgGCCGAGAAGAGCCTGATCGTAG- 3' | Proline |
| 23 | 5'-TCGATTACAACCTGCCCTGCagcGCCGAGAAGAGCCTGATCGTAG- 3' | Serine |
| 24 | 5'-TCGATTACAACCTGCCCTGCtgcGCCGAGAAGAGCCTGATCGTAG-3' | Cysteine |
| 25 | 5'-TCGATTACAACCTGCCCTGCtatGCCGAGAAGAGCCTGATCGTAG-3' | Tyrosine |
| 26 | 5'-TCGATTACAACCTGCCCTGCaacGCCGAGAAGAGCCTGATCGTAG- 3' | Asparagine |
| 27 | 5'-TCGATTACAACCTGCCCTGCcagGCCGAGAAGAGCCTGATCGTAG- 3' | Glutamine |
| 28 | 5'-TCGATTACAACCTGCCCTGCgatGCCGAGAAGAGCCTGATCGTAG-3' | Aspartic acid |
| 29 | 5'-TCGATTACAACCTGCCCTGCgaaGCCGAGAAGAGCCTGATCGTAG- | Glutamic acid |
| | 3' | |
| 30 | 5'-TCGATTACAACCTGCCCTGCaaaGCCGAGAAGAGCCTGATCGTAG- 3' | Lysine |
| 31 | 5'-TCGATTACAACCTGCCCTGCcgtGCCGAGAAGAGCCTGATCGTAG-3' | Arginine |
| 32 | 5'-TCGATTACAACCTGCCCTGCcatGCCGAGAAGAGCCTGATCGTAG-3' | Histidine |

A total of 18 kinds of plasmids prepared above were transformed by homologous recombination using the MBX184 strain as a parent strain and plated on a solid LB medium containing ampicillin (100 mg/L) to prepare strains introduced with mutated propionaldehyde dehydrogenase. The names of the prepared plasmids and the introduced strains are shown in Table 9 below. That is, in each plasmid listed in Table 9, as the pFS-pduP(WT) plasmid of Example 1 above, the gene encoding the propionaldehyde dehydrogenase variants was inserted so as to be expressed as one operon under the control of the trc promoter together with glycerol dehydratase derived from *Klebsiella pneumoniae* and PHA synthase derived from *Ralstonia eutropha.* Additionally, each plasmid contains the *lacl* gene derived from pSE380 (Thermo Fisher Scientific Inc., Waltham, MA, USA), the ampicillin resistance gene (*ampR*)*,* and the origin of replication (Ori).

**[Table 9-**

| Substituted Amino Acids | Name of Plasmids | Name of Strains |
|---|---|---|
| Glycine | pFS-pduP(I272G) | 184/pFS-pduP(I272G) |
| Alanine | pFS-pduP(I272A) | 184/pFS-pduP(I272A) |
| Valine | pFS-pduP(I272V) | 184/pFS-pduP(I272V) |
| Leucine | pFS-pduP(I272L) | 184/pFS-pduP(I272L) |
| Methionine | pFS-pduP(I272M) | 184/pFS-pduP(I272M) |
| Phenylalanine | pFS-pduP(I272F) | 184/pFS-pduP(I272F) |
| Tryptophan | pFS-pduP(I272W) | 184/pFS-pduP(I272W) |
| Proline | pFS-pduP(I272P) | 184/pFS-pduP(I272P) |
| Serine | pFS-pduP(I272S) | 184/pFS-pduP(I272S) |
| Cysteine | pFS-pduP(I272C) | 184/pFS-pduP(I272C) |
| Tyrosine | pFS-pduP(I272Y) | 184/pFS-pduP(I272Y) |
| Asparagine | pFS-pduP(I272N) | 184/pFS-pduP(I272N) |
| Glutamine | pFS-pduP(I272Q) | 184/pFS-pduP(I272Q) |
| Aspartic acid | pFS-pduP(I272D) | 184/pFS-pduP(I272D) |
| Glutamic acid | pFS-pduP(I272E) | 184/pFS-pduP(I272E) |
| Lysine | pFS-pduP(I272K) | 184/pFS-pduP(I272K) |
| Arginine | pFS-pduP(I272R) | 184/pFS-pduP(I272R) |
| Histidine | pFS-pduP(I272H) | 184/pFS-pduP(I272H) |

To evaluate the P3HP production ability of the strains containing the propionaldehyde dehydrogenase variants prepared above, a total of 20 strains including the control 184/pFS-pduP(WT) and the 184/pFS-pduP(mt)-2101 strain were cultured in the same manner as in Example 3, and the P3HP production ability was analyzed. The results are shown in Table 10 below.

**[Table 10]**

| Strain | OD (600 nm) | DCW(g/L) | P3HP Production (%) | P3HP Titer (mg/L) |
|---|---|---|---|---|
| 184/pFS-pduP(WT) | 2.89 | 0.79 | 5.92 | 46.6 |
| 184/pFS-pduP(mt)- 2101 | 3.43 | 0.92 | 8.78 | 80.7 |
| 184/pFS-pduP(I272V) | 3.37 | 0.83 | 7.91 | 65.4 |
| 184/pFS-pduP(I272Y) | 2.92 | 0.79 | 6.11 | 48.3 |
| 184/pFS-pduP(I272N) | 3.45 | 1.04 | 9.18 | 95.1 |
| 184/pFS-pduP(I272Q) | 3.07 | 0.86 | 7.14 | 61.3 |

As a result, it was confirmed that all novel variants in which the amino acid at position 272 of propionaldehyde dehydrogenase was substituted with an amino acid selected from valine, threonine, tyrosine, asparagine, and glutamine showed an increase in P3HP production as compared to the control group.

Specifically, the 184/pFS-pduP(I272N) strain, in which the amino acid at position 272 of propionaldehyde dehydrogenase was substituted with asparagine, showed an increase in P3HP production by 55% and titer by up to 104% as compared to the control group 184/pFS-pduP(WT).

Based on the results, it was confirmed that the position 272 of propionaldehyde dehydrogenase is an important position for P3HP production.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the present disclosure is defined by the appended claims rather than by the description preceding them, and therefore all changes and modifications that fall within metes and bounds of the claims, or equivalents of such metes and bounds are therefore intended to be embraced by the claims.

## Claims

1. A variant polypeptide having propionaldehyde dehydrogenase activity in which the amino acid corresponding to position 272 of SEQ ID NO: 1 is substituted with another amino acid.

2. The variant polypeptide of claim 1, wherein the amino acid corresponding to position 272 of SEQ ID NO: 1 is substituted with any one selected from the group consisting of glycine, alanine, arginine, valine, leucine, methionine, threonine, asparagine, glutamine, proline, serine, tryptophan, phenylalanine, histidine, cysteine, tyrosine, lysine, aspartate, and glutamic acid.

3. The variant polypeptide of claim 1, wherein the variant polypeptide comprises an amino acid sequence selected from the group consisting of SEQ ID NOS: 3 to 7 and SEQ ID NOS: 33 to 46.

4. A polynucleotide encoding the variant polypeptide of any one of claims 1 to 3.

5. A microorganism comprising any one or more of the variant polypeptide of any one of claims 1 to 3, a polynucleotide encoding the variant polypeptide, and a vector containing the polynucleotide.

6. The microorganism of claim 5, wherein the microorganism has an increased production ability of 3HP-CoA or derivatives thereof, as compared to a microorganism comprising a polypeptide of SEQ ID NO: 1 or a polynucleotide encoding the polypeptide.

7. The microorganism of claim 5, which is a microorganism of the genus *Escherichia.*

8. The microorganism of claim 7, wherein the microorganism of the genus *Escherichia* is *Escherichia coli.*

9. A method for producing 3HP-CoA or derivatives thereof, comprising culturing the microorganism of claim 5 in a medium.

10. The method of claim 9, further comprising a step of recovering a target material from the cultured microorganism, a culture product of the microorganism, a fermented product of the microorganism, or the culture medium.

11. A composition for producing 3HP-CoA or derivatives thereof, comprising the variant polypeptide of claim 1; a microorganism comprising any one or more of the variant polypeptide, a polynucleotide encoding the variant polypeptide, and a vector containing the polynucleotide; or a combination thereof.

12. Use of the variant polypeptide of claim 1; or a microorganism comprising any one or more of the variant polypeptide, a polynucleotide encoding the variant polypeptide, and a vector containing the polynucleotide for the production of 3HP-CoA or derivatives thereof.
